# FASCICULE DE BREVET EUROPEEN

(11) **EP 2 188 380 B1**
(45) Date de publication et mention de la délivrance du brevet: **11.05.2011**
(21) Numéro de dépôt: 08837603.3
(22) Date de dépôt: 17.09.2008
(51) Int. Cl.: C12P 7/44, C12P 7/64, C07C 57/03, C07C 69/716, C07C 227/08, C07C 229/08, C10G 9/36, C07C 67/00, C07C 51/00, C07C 227/06

(54) **Procédé de coproduction de 7-oxoheptanoate de méthyle et d'acide undécylénique a partir d'acide ricinoléique**
Verfahren zur gleichzeitigen Herstellung von Methyl 7-Oxoheptanoat und Undecylensäure aus Ricinoleinsäure
Method for the coproduction of methyl 7-oxoheptanoate and undecylenic acid from ricinoleic acid

(30) Priorité: 20.09.2007 FR 0757696
(43) Date de publication de la demande: 26.05.2010
(73) Titulaire: ARKEMA FRANCE, 92700 Colombes (FR)
(72) Inventeur: DUBOIS, Jean-Luc, F-69390 Millery (FR)
(74) Mandataire: Sauvageot, Olivier Roger
(86) Numéro de dépôt international: PCT/FR2008/051666
(87) Numéro de publication internationale: WO 2009/047446

(56) Documents cités:
- GB-A- 825 932
- US-A1- 2002 061 566
- NAUGHTON F C: "PRODUCTION CHEMISTRY AND COMMERCIAL APPLICATIONS OF VARIOUS CHEMICALS FROM CASTOR OIL" JOURNAL OF THE AMERICAN OIL CHEMISTS' SOCIETY, vol. 51, no. 3, 1974, pages 65-71, XP002469296 ISSN: 0003-021X
- FABRITIUS D ET AL: "Bioconversion of sunflower oil, rapeseed oil and ricinoleic acid by Candida tropicalis M25" APPLIED MICROBIOLOGY AND BIOTECHNOLOGY, vol. 50, no. 5, novembre 1998 (1998-11), pages 573-578, XP002469297 ISSN: 0175-7598

## Description

L'invention vise un procédé de transformation d'acide ricinoléique incluant une étape de fermentation de cet acide, en vue d'obtenir, notamment du 7-oxoheptanoate de méthyle et de l'acide 10-undécylénique (encore dénommé acide undécyl-10-énoique, et par la suite plus simplement appelé acide undécylénique).

La fermentation de l'acide ricinoléique conduit au diacide 7-hydroxy-9-octadécènedioique.

Les diacides ou diesters insaturés les plus connus sont ceux comportant des chaînes comportant de 4 à 6 atomes de carbone tels que les acides en C4, acides maléique et fumarique, les acides en C5, acides citraconique, mesaconique et itaconique et les acides en C6, les acides 2-méthylène glututarique et muconique. En revanche en ce qui concerne les diacides à chaîne longue, les seuls ayant une certaine importance sont des dimères généralement obtenus par condensation d'acides carboxyliques insaturés. Les propriétés, synthèses et utilisations de ces diacides sont décrites dans l'Encyclopédie Kirk-Othmer Vol. A8 pages 533-536.

Les diacides saturés sont obtenus industriellement selon différentes méthodes, mais qui toutes présentent certains inconvénients. Un large panorama de ces méthodes est développé sous la référence précédente aux pages 523-536.

On peut y distinguer les méthodes par dégradation telles que l'ozonolyse ou l'oxydation des acides gras végétaux.

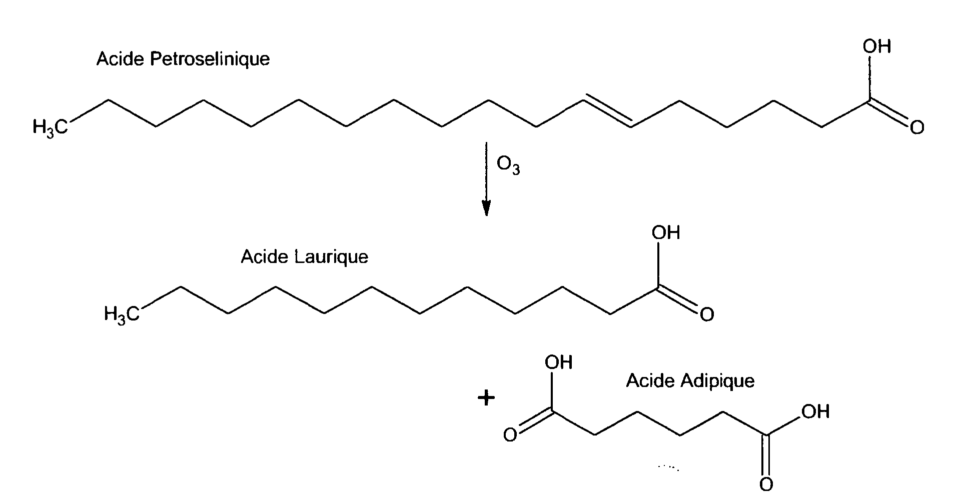

L'ozonolyse de l'acide oléique, de l'acide pétrosélinique et de l'acide érucique permet de produire respectivement les diacides à 9, 6 et 13 atomes de carbone selon le processus réactionnel ci-dessus pour l'acide pétrosélinique.

Un autre exemple est le clivage de l'acide ricinoléique par action de la soude à une température supérieure à 180°C. Cette méthode utilisée industriellement permet d'obtenir le diacide à 10 atomes de carbone. La même méthode, telle qu'illustrée dans le schéma ci-dessous peut être appliquée à l'acide lesquérolique et conduit à la formation d'un diacide à 12 atomes de carbone. Cette méthode présente l'avantage d'utiliser des matières premières renouvelables mais est limitée essentiellement au diacide en C10, l'acide lesquérolique étant encore peu répandu, et donc cette méthode est relativement peu utilisée.

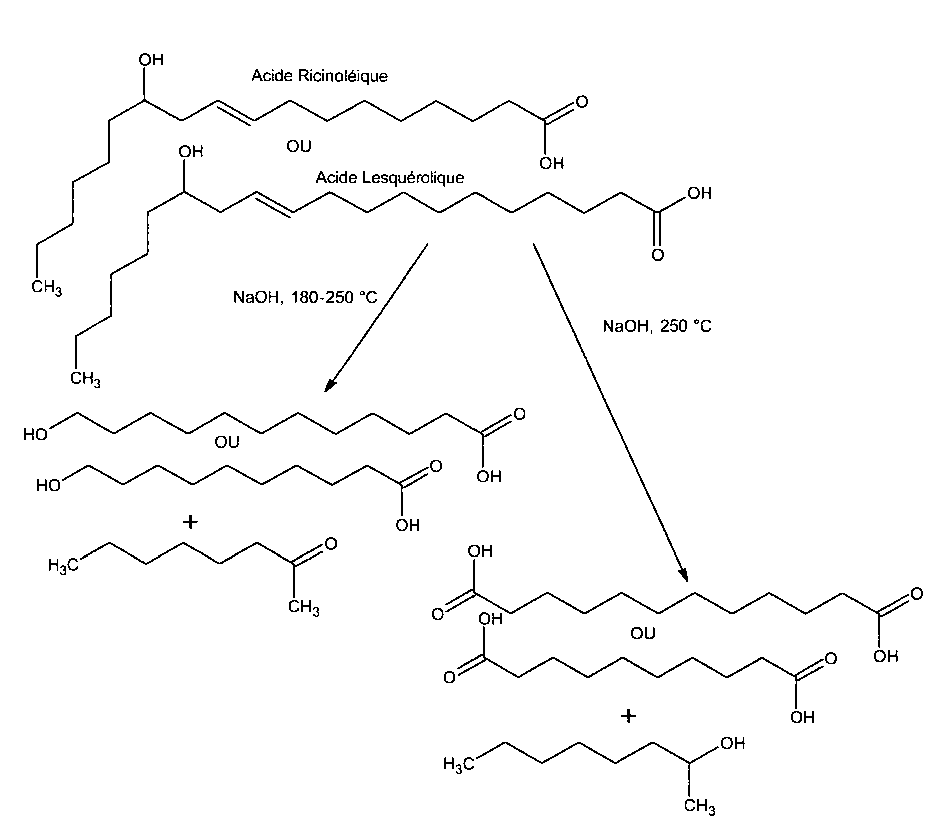

On peut citer aussi la dégradation oxydante des acides monocarboxyliques par action de N₂O₄. L'oxydation de l'acide stéarique permet d'obtenir un mélange d'acide sébacique et d'acide caprylique ; à partir d'acide palmitique on peut obtenir l'acide subérique.

Il est également possible d'obtenir des diacides à partir de molécules de plus petites tailles en utilisant des techniques variantes de la carbonylation.

Enfin on peut citer la fermentation par une levure, un champignon ou une bactérie de substrats hydrocarbonés paraffines, alcools, acides ou esters gras saturés ou non qui permet d'obtenir une oxydation des composés du substrat. Cette méthode est bien connue. Elle est notamment illustrée dans l'article de W. H. Eschenfeldt et al. « Transformation of fatty Acids Catalyzed by Cytochrome P450 Monooxygenase Enzymes of Candida tropicalis » et les brevets FR 2,445,374, US 4,474,882, US 3,823,070, US 3,912,586, US 6,660,505, US 6,569,670 et 5,254,466. Elle permet d'obtenir de nombreux diacides de longueur de chaîne variable. La production de diacide 9-octadécènedioique par fermentation d'acide oléique et d'alcool oléique est décrite dans la publication de Zu-Hua Yi et Hans-Jürgen Rehm dans Appl. Microbiol. Biotechnol. (1989) 30 : 327-331.

La littérature est relativement pauvre en documents illustrant la formation de diacides insaturés hydroxylés. La publication de D. Fabritius, H.J. Schäfer et A Steinbüchel dans Appl. Microbiol. Biotechnol. (1996) 45 : 342-348, illustre la formation de 3-Hydroxy-9-octadécènedioique, par fermentation de l'acide oléique avec une souche mutante de *Candida Tropicalis.* La mutation de la souche de C tropicalis DSM 3152 avec du N-méthyl-N-nitro-N'-nitrosoguanidine a conduit à un mutant M25 qui permet d'obtenir une concentration 1.8 fois plus élevée de diacide hydroxylé qu'avec la souche mère.

Le brevet US 6 569 670 décrit un procédé de fermentation pour la production de diacides. L'utilisation d'acides gras naturels est décrite colonne 6 ligne 25. Notamment l'acide ricinoléique, ses esters et les triglycérides ayant une forte teneur en acide ricinoléique sont décrits quoique non illustrés.

L'évolution actuelle en matière d'environnement conduit dans les domaines de l'énergie et de la chimie à privilégier l'exploitation des matières premières naturelles provenant d'une source renouvelable. C'est ainsi que les acides/esters gras extraits d'huiles ou de graisse d'origine végétale ou animale, et donc renouvelables, deviennent des matières premières particulièrement intéressantes.

Un exemple de procédé industriel utilisant un acide gras comme matière première est celui de la fabrication à partir de l'acide ricinoléique extrait de l'huile de ricin, de l'acide amino-11-undécanoique, qui est à la base de la synthèse du Rilsan®. Ce procédé est décrit dans l'ouvrage « Les Procédés de Pétrochimie » de A. Chauvel et al. paru aux Editions TECHNIP (1986). L'acide amino-11-undécanoique est obtenu en plusieurs étapes. La première consiste en une méthanolyse de l'huile de ricin en milieu basique produisant le ricinoléate de méthyle qui est ensuite soumis à une pyrolyse pour obtenir d'une part l'heptanaldéhyde et d'autre part l'undécylénate de méthyle. Ce dernier est passé en forme acide par hydrolyse. Ensuite l'acide formé est soumis à une hydrobromuration pour donner l'acide w-bromé d'où l'on passe par amination à l'acide amino-11-undécanoique.

Ce procédé conduit à la co-production de l'acide amino-11-undécanoique et d'heptanaldéhyde qu'il convient de valoriser séparément. L'heptanaldéhyde trouve des applications dans des marchés de parfumerie, ou chimie fine, ou encore doit être transformé en acide heptanoïque par oxydation pour des applications en huiles, plastifiants, peintures ou encore doit être transformé en heptanol pour des applications en plastifiants, hygiène et santé. D'autre part, l'heptanaldéhyde n'a qu'un marché limité, et est principalement transformé notamment en acide heptanoïque pour être valorisé.

L'huile de ricin contient environ 85 % d'acide ricinoléique. Dans le procédé conventionnel de production d'acide 10-undécylénique, les autres acides gras présents dans l'huile de ricin ne sont pas craqués et conduisent à une coupe appelée Estérol comprenant des composés à faible valeur ajoutée.

Ces marchés peuvent être très fluctuants et il est souhaitable de trouver un procédé qui puisse permettre d'éviter la production d'heptanaldéhyde, tout en favorisant la formation d'autres composés, utilisables industriellement par exemple dans la production de polymères techniques, ou en pharmacie.

Dans l'industrie pharmaceutique, le 7-oxoheptanoate de méthyle est particulièrement utile, en particulier lors de la synthèse de prostanoïdes tels que le 2-(6-méthoxycarbonylhexyl)-cyclopent-2-en-1-one, (Bosone, Enrico ; Synthesis 1983 (11) p942-4) ou lors de la synthèse de prostaglandines telles que le Mexiprostil®, il est nécessaire de disposer de 7-oxoheptanoate de méthyle. (Kolb, M. ; Tetrahedron Letters 1988 V29 (51) P6769-72).

De plus, l'acide undécylénique est également utile dans le domaine de l'industrie pharmaceutique, où il est utilisé comme principe actif dans des compositions destinées au traitement des mycoses.

Il est donc nécessaire de trouver un type de procédé qui permet d'obtenir à la fois du 7-oxoheptanoate de méthyle et de l'acide undécylénique et qui, en outre, utilise une matière renouvelable d'origine naturelle, l'huile de ricin.

L'invention a donc pour objet un procédé de transformation de l'huile de ricin.

Selon l'invention, ledit procédé comprend les étapes successives suivantes :
a) la fermentation de l'acide ricinoléique par une bactérie, une levure ou un champignon pour obtenir le diacide-7-hydroxy-9-octadécènedioique,
b) l'estérification du diacide-7-hydroxy-9-octadécènedioique obtenu à l'étape a) pour former le diester méthylique correspondant,
c) le craquage à haute température du produit obtenu à l'étape b) pour former un mélange d'undécylénate de méthyle et de 7-oxoheptanoate de méthyle,
d) la séparation de l'undécylénate de méthyle et du 7-oxoheptanoate de méthyle obtenus à l'étape c),
e) l'hydrolyse de l'undécylénate de méthyle obtenu à l'étape d) pour former l'acide undécylénique.

Selon le procédé de l'invention, du 7-oxoheptanoate de méthyle, est obtenu à l'issue de l'étape d), et de l'acide undécylénique est obtenu à l'issue de l'étape e).

Dans des modes de réalisation préférés de l'invention, l'acide undécylénique obtenu à l'étape e) peut être transformé en acide amino-11 undécanoïque et le 7-oxoheptanoate de méthyle obtenu à l'étape d) peut être transformé en acide amino-7-heptanoïque.

Ces composés qui sont des aminoacides sont particulièrement utiles dans l'industrie chimique et notamment celles des polymères, telle que la production de polyamides, ou de polymères techniques.

En conséquence, le procédé objet de l'invention peut comprendre en outre les étapes suivantes :
f) la réaction de l'acide undécylénique obtenu à l'étape e) avec HBr pour former l'acide 11-bromo-undécylénique,
g) la réaction de l'acide 11-bromo-undécylénique obtenu à l'étape f) avec l'ammoniaque pour former l'acide amino-11-undécanoïque.

Le procédé objet de l'invention peut également comprendre une étape d'amination réductrice du 7-oxoheptanoate de méthyle obtenu à l'étape d) suivie d'une étape d'hydrolyse pour former l'acide amino-7-heptanoïque.

L'acide amino-7-heptanoique est un monomère remarquable. En effet, ce monomère a peu tendance à ce cycliser et le polyamide 7, issu de l'amino-7-heptanoïque a un point de fusion plus élevé que le polyamide 6.

De préférence, l'acide ricinoléique est issu des huiles végétales extraites principalement du ricin, mais aussi de certaines algues, et de plantes génétiquement modifiées ou non comme le lin ou le colza.

L'acide ricinoléique mis en oeuvre à l'étape a) peut provenir d'huile de ricin. L'huile de ricin contient environ 85 % d'acide ricinoléique, et un faible pourcentage d'autres acides gras.

Lorsque de l'huile de ricin est mise en oeuvre à l'étape a) du procédé défini ci-dessus, les acides gras autres que l'acide ricinoléique contenus dans cette huile sont transformés, après l'étape c) de craquage à haute température en diacides en C18, C16 et C20 qui trouvent eux aussi des applications dans la production des polymères techniques.

Dans la suite de la présente description on utilisera pour la clarté de l'exposé le terme acide pour désigner indifféremment l'acide et l'ester ou le triglycéride. En effet dans le procédé de l'invention, on peut traiter l'acide ricinoléique soit sous sa forme acide soit sous sa forme ester soit sous sa forme triglycéride. Le passage d'une forme à l'autre s'effectue par alcoolyse, estérification, transestérification ou hydrolyse.

Dans le procédé de l'invention on utilise des acides ou esters gras d'origine naturelle, c'est-à-dire contenus dans des huiles ou des graisses. Ces dernières sont en fait constituées à côté de l'ester ou de l'acide entrant dans la réaction d'un mélange d'esters ou acides de formules voisines. A titre d'exemple l'huile de ricin contient outre l'acide ricinoléique à la fois de l'acide oléique et l'acide linoléique, de l'acide palmitique, de l'acide stéarique et de l'acide arachidique. La présence de ces acides saturés, mono-insaturés, di-insaturés ou poly-insaturés n'a pas de conséquence importante sur le déroulement du procédé dans la mesure où lors de l'étape de craquage une séparation des produits est effectuée.

La première étape du procédé objet de l'invention, l'étape a) est réalisée par fermentation en présence d'un micro-organisme c'est-à-dire au moyen de toute bactérie, champignon ou levure permettant l'oxydation de l'acide, triglycéride ou ester gras de la charge.

L'étape de fermentation peut être réalisée en présence d'un micro-organisme contenant une enzyme permettant d'obtenir le diacide-7-hydroxy 9-octadécènedioique.

On peut indifféremment fermenter un acide gras, un ester gras et un triglycéride. En effet, les micro-organismes sont aussi capables de métaboliser les alcools et le glycérol.

L'étape b) du procédé objet de l'invention consiste en l'estérification du diacide-7-hydroxy-9-octadécènedioique obtenu à l'étape a) pour former le diester méthylique correspondant. De préférence, cette étape d'estérification a lieu en présence d'un excès de méthanol et d'un catalyseur, par exemple un catalyseur acide.

L'étape c) de craquage à haute température du produit obtenu à l'étape b) pour former un mélange d'undécylénate de méthyle et de 7-oxoheptanoate de méthyle, a lieu de préférence à une température allant de 400°C à 600°C.

L'étape d) de séparation est une étape classique pour l'homme du métier et ne sera donc pas décrite en détail. A titre d'exemple, la séparation peut consister en une distillation.

L'étape e) d'hydrolyse a lieu de préférence à une température proche de la température ambiante, en présence de soude ; mais peut aussi être effectuée à 80°C et sous pression par exemple.

L'étape f) consiste en une hydrobromuration, réalisée de préférence en présence d'un solvant, par exemple le toluène et d'un initiateur de radicaux libres, tel que le peroxyde de benzoyle.

Enfin, l'étape g) d'amination se déroule de préférence en présence d'un grand excès d'une solution d'ammoniaque.

L'amination réductrice de l'acide 7-oxoheptanoique obtenu à l'étape c) pour former l'acide amino-7-heptanoïque peut être effectuée selon de nombreuses méthodes, catalytiques ou enzymatiques, et par exemple selon la méthode décrite dans la demande de brevet US5,973,208.

Dans un procédé alternatif, de l'acide lesquérolique est utilisé en remplacement de l'acide ricinoléique.

Ainsi, il est exposé un procédé de transformation de l'acide lesquérolique, comprenant les étapes successives suivantes :
a) la fermentation de l'acide lesquérolique par une bactérie, une levure ou un champignon pour obtenir le diacide-7-hydroxy 11-eicosènedioique,
b) l'estérification du diacide-7-hydroxy 11-eicosènedioique obtenu à l'étape a) pour former le diester méthylique correspondant,
c) le craquage à haute température du produit obtenu à l'étape b) pour former un mélange de 12-tridécénoate de méthyle et de 7-oxoheptanoate de méthyle,
d) la séparation du 12-tridécénoate de méthyle et du 7-oxoheptanoate de méthyle obtenus à l'étape c),
e) l'hydrolyse du 12-tridécénoate de méthyle obtenu à l'étape d) pour former l'acide 12-tridécénoique.

Ce procédé peut comprendre en outre une étape d'amination réductrice du 7-oxoheptanoate de méthyle obtenu à l'étape d) suivi d'une étape d'hydrolyse pour former l'acide amino-7-heptanoïque.

Les exemples suivants illustrent, sans le limiter, le procédé objet de l'invention, mettant en oeuvre de l'acide ricinoléique.

### EXEMPLES

### Exemple 1 : Etape a) du procédé, fermentation de l'acide ricinoléique.

Dans cet exemple une souche de levure est cultivée dans un milieu contenant de l'acide ricinoléique. Le mélange est ensuite stérilisé à 120°C pendant 15 minutes. La culture est maintenue à 30°C. Une solution de soude est ajoutée en continu pour maintenir le pH de 7 à - 7,5. Après 48 heures de culture, le diacide insaturé hydroxylé est récupéré par extraction au diéthylether. En mettant en oeuvre ce mode opératoire, on peut obtenir le diacide 7-Hydroxy-9-octadécènedioique.

### Exemple 2 : Etape b) du procédé, estérification du diacide 7-Hydroxy-9-octadécènedioique.

Le diacide produit est estérifié en diester méthylique par réaction en présence d'un catalyseur acide et de méthanol. 1 g de diacide est mis en présence de méthanol en présence d'un catalyseur acide hétérogène zircone sulfatée obtenue chez Wako. Le mélange réactionnel est chauffé à 150°C jusqu'à l'équilibre puis le méthanol en excès est distillé. Ces conditions expérimentales permettent d'obtenir le diester méthylique du diacide 7-Hydroxy-9-octadécènedioique.

### Exemple 3 : Etape c) du procédé, craquage thermique du diester.

Le craquage thermique du diester méthylique est effectué à 500°C dans un four tubulaire avec un temps de séjour de 15 secondes. Après vaporisation du diester à 260°C, il est mélangé avec de la vapeur d'eau chauffée à 600°C. Le flux issu de cette réaction est tout d'abord condensé dans une série d'échangeurs où la température est abaissée à environ 150°C. L'eau est alors séparée par décantation. Les produits formés sont fractionnés dans des colonnes à distiller successives. Avec ces conditions expérimentales, on peut récupérer ainsi les fractions de l'ester méthylique de l'acide undécylénique, de l'ester de l'acide 7-oxoheptanoique, des diesters autres que celui issu de l'acide ricinoléique. Le produit non converti peut être recyclé à l'entrée du réacteur après séparation.

Les étapes d), e) et f) du procédé objet de l'invention sont des étapes classiques pour l'homme du métier, et ne sont donc pas illustrées.

### Exemple 4 : Amination réductrice de l'ester méthylique de l'acide 7-oxoheptanoique.

Dans un autoclave équipé d'un agitateur magnétique on introduit 1 g de catalyseur Chromite de cuivre, 8 g de l'ester méthylique de l'acide 7-oxoheptanoique, 30 g d'éthanol et 30 g d'ammoniac. Ensuite on introduit de l'hydrogène à une pression de 80 bars. L'autoclave est ensuite chauffé à 150°C sur 1 heure, et maintenu à cette température pendant 2 heures. Après filtration pour éliminer le catalyseur, et élimination du solvant, le produit est analysé. Ces conditions expérimentales permettent d'obtenir l'ester méthylique de l'acide 7-aminoheptanoique. Ce dernier peut ensuite être hydrolysé pour former l'acide 7-aminoheptanoique. L'hydrolyse est effectuée à 25°C dans un réacteur à double enveloppe, avec un temps de séjour de 30 minutes. Le méthanol peut être recyclé après purification à l'étape d'estérification.

## Revendications

1. Procédé de transformation de l'acide ricinoléique, comprenant les étapes successives suivantes :
a) la fermentation de l'acide ricinoléique par une bactérie, une levure ou un champignon pour obtenir le diacide-7-hydroxy-9-octadécènedioique,
b) l'estérification du diacide-7-hydroxy-9-octadécènedioique obtenu à l'étape a) pour former le diester méthylique correspondant,
c) le craquage à haute température du produit obtenu à l'étape b) pour former un mélange d'undécylénate de méthyle et de 7-oxoheptanoate de méthyle,
d) la séparation de l'undécylénate de méthyle et du 7-oxoheptanoate de méthyle obtenus à l'étape c),
e) l'hydrolyse de l'undécylénate de méthyle obtenu à l'étape d) pour former l'acide undécylénique.

2. Procédé selon la revendication 1, **caractérisé en ce qu'**il comprend en outre les étapes suivantes :
f) la réaction de l'acide undécylénique obtenu à l'étape e) avec HBr pour former l'acide 11-bromo-undécylénique,
g) la réaction de l'acide 11-bromo-undécylénique obtenu à l'étape f) avec l'ammoniaque pour former l'acide amino-11-undécanoïque.

3. Procédé selon la revendication 1 ou 2, **caractérisé en ce qu'**il comprend en outre une étape d'amination réductrice du 7-oxoheptanoate de méthyle obtenu à l'étape d) suivi d'une étape d'hydrolyse pour former l'acide amino-7-heptanoïque.

## Claims

1. Process for the conversion of ricinoleic acid comprising the following successive stages:
a) the fermentation of ricinoleic acid by a bacterium, a yeast or a fungus, in order to obtain 7-hydroxy-9-octadecenedioic diacid,
b) the esterification of the 7-hydroxy-9-octadecenedioic diacid obtained in stage a), in order to form the corresponding methyl diester,
c) the high-temperature cracking of the product obtained in stage b), in order to form a mixture of methyl undecylenate and methyl 7-oxoheptanoate,
d) the separation of the methyl undecylenate and methyl 7-oxoheptanoate obtained in stage c),
e) the hydrolysis of the methyl undecylenate obtained in stage d), in order to form undecylenic acid.

2. Process according to Claim 1, **characterized in that** it additionally comprises the following stages:
f) the reaction of the undecylenic acid obtained in stage e) with HBr, in order to form 11-bromoundecylenic acid,
g) the reaction of the 11-bromoundecylenic acid obtained in stage f) with ammonia, in order to form 11-aminoundecanoic acid.

3. Process according to Claim 1 or 2, **characterized in that** it additionally comprises a stage of reductive amination of the methyl 7-oxoheptanoate obtained in stage d), followed by a hydrolysis stage, in order to form 7-aminoheptanoic acid.

## Patentansprüche

1. Verfahren zur Umwandlung von Ricinolsäure mit den folgenden aufeinanderfolgenden Schritten:
a) Fermentation von Ricinolsäure mit einem Bakterium, einer Hefe oder einem Pilz zum Erhalt von 7-Hydroxy-9-octadecendisäure,
b) Veresterung der in Schritt a) erhaltenen 7-Hydroxy-9-octadecendisäure zur Bildung des entsprechenden Dimethylesters,
c) Spaltung des in Schritt b) erhaltenen Produkts bei hoher Temperatur zur Bildung eines Gemischs von Undecylensäuremethylester und 7-Oxoheptansäuremethylester,
d) Trennung des in Schritt c) erhaltenen Undecylensäuremethylesters und 7-Oxoheptansäuremethylesters,
e) Hydrolyse des in Schritt d) erhaltenen Undecylensäuremethylesters zur Bildung von Undecylensäure.

2. Verfahren nach Anspruch 1, **dadurch gekennzeichnet, daß** es außerdem die folgenden Schritte umfaßt:
f) Umsetzung der in Schritt e) erhaltenen Undecylensäure mit HBr zur Bildung von 11-Bromundecylensäure,
g) Umsetzung der in Schritt f) erhaltenen 11-Bromundecylensäure mit wäßrigem Ammoniak zur Bildung von 11-Aminoundecylensäure.

3. Verfahren nach Anspruch 1 oder 2, **dadurch gekennzeichnet, daß** es außerdem einen Schritt der reduktiven Aminierung des in Schritt d) erhaltenen 7-Oxoheptansäuremethylesters gefolgt von einem Hydrolyseschritt zur Bildung von 7-Aminoheptansäure umfaßt.
